# EUROPÄISCHE PATENTSCHRIFT

(11) **EP 2 038 294 B1**
(45) Veröffentlichungstag und Bekanntmachung des Hinweises auf die Patenterteilung: **08.12.2010**
(21) Anmeldenummer: 07765033.1
(22) Anmeldetag: 29.06.2007
(51) Int. Cl.: C07J 53/00, A61K 31/58, A61P 5/42, A61P 5/34

(54) **18-METHYL-19-NOR-ANDROST-4-EN-17,17-SPIROETHER (18-METHYL-19-NOR-20- SPIROX-4-EN-3-ONE), SOWIE DIESE ENTHALTENDE PHARMAZEUTISCHE PRÄPARATE**
18-METHYL-19-NOR-ANDROST-4-EN-17,17-SPIROETHER (18-METHYL-19-NOR-20- SPIROX-4-EN-3-ONE) AND PHARMACEUTICAL PREPARATIONS CONTAINING THE SAME
18-METHYL-19-NOR-ANDROST-4-EN-17,17-SPIROÉTHERS (18-METHYL-19-NOR-20- SPIROX-4-EN-3-ONE) ET PRÉPARATIONS PHARMACEUTIQUES LES CONTENANT

(30) Priorität: 29.06.2006 DE 102006030416
(43) Veröffentlichungstag der Anmeldung: 25.03.2009
(73) Patentinhaber: Bayer Schering Pharma Aktiengesellschaft, 13353 Berlin (DE)
(72) Erfinder: BOHLMANN, Rolf, 14055 Berlin (DE); KUHNKE, Joachim, 14482 Potsdam (DE); HÜBNER, Jan, 10317 Berlin (DE); GALLUS, Norbert, 12099 Berlin (DE); MENGES, Frederik, 69198 Schriesheim (DE); BORDEN, Steffen, 13355 Berlin (DE); MUHN,Hans-Peter, 13465 Berlin (DE); PRELLE, Katja, 13465 Berlin (DE)
(86) Internationale Anmeldenummer: PCT/EP2007/005893
(87) Internationale Veröffentlichungsnummer: WO 2008/000521

(56) Entgegenhaltungen:
- EP-A- 0 150 157
- EP-A- 1 148 061
- WO-A-2006/072467
- DE-A1- 3 111 950
- US-A- 3 798 213
- NICKISCH K ET AL: "Aldosterone antagonists. 4. Synthesis and activities of steroidal 6,6-ethylene-15,16-methylene 17-spirolactones" JOURNAL OF MEDICINAL CHEMISTRY, AMERICAN CHEMICAL SOCIETY. WASHINGTON, US, Bd. 34, 1991, Seiten 2464-2468, XP002375413 ISSN: 0022-2623
- CASALS-STENZEL, J. ET AL: "The renal action of spirorenone and other 6.beta.,7.beta.; 15.beta.,16.beta.-dimethylene-17-spirolact ones, a new type of steroidal aldosterone antagonists" ARZNEIMITTEL-FORSCHUNG , 34(3), 241-6 CODEN: ARZNAD; ISSN: 0004-4172, 1984, XP002457524

## Beschreibung

Die vorliegende Erfindung betrifft 18-Methyl-15β,16β-methylen-19-nor-20-spirox-4-en-3-one [entsprechen [(17S)-Spiro[18a-homo-15α,16α-dihydro-3'H-cyclopropa[15,16] estr-4-en-17,2'-perhydrofuran]-3-onen] der allgemeinen Formel I 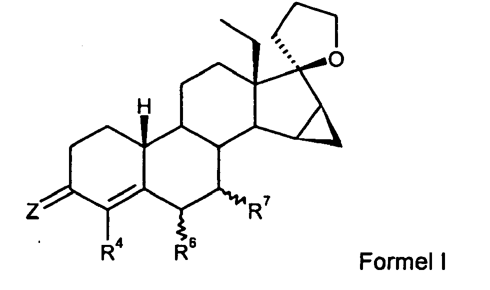 worin
Z ein Sauerstoffatom, zwei Wasserstoffatome, eine Gruppierung =NOR oder =NNHSO₂R, wobei R ein Wasserstoffatom oder eine gerad- oder verzweigtkettige Alkylgruppe mit 1 bis 4 bzw. 3 bis 4 Kohlenstoffatomen ist,
R⁴ ein Wasserstoffatom, ein Halogenatom oder eine Trifluormethylgruppe, und R⁶ und/oder R⁷ α- oder β-ständig sein kann und R⁶ und R⁷ unabhängig voneinander ein Wasserstoffatom oder eine gerad- oder verzweigtkettige Alkylgruppe mit 1 bis 4 bzw. 3 bis 4 Kohlenstoffatomen oder eine gerad- oder verzweigtkettige Alkenylgruppe mit 2 bis 4 bzw. 3 bis 4 Kohlenstoffatomen oder eine gesättigte Cycloalkylgruppe mit 3 bis 5 Kohlenstoffatomen oder gemeinsam eine Methylengruppe oder eine Doppelbindung,
bedeuten.
Z steht vorzugsweise für ein Sauerstoffatom.
Im Falle, daß Z für eine Gruppierung =NOR oder =NNHSO₂R steht, ist R vorzugsweise ein Wasserstoffatom.

Für eine gerad- oder verzweigtkettige Alkylgruppe mit 1 bis 4 bzw. 3 bis 4 Kohlenstoffatomen kommt eine Methyl-, Ethyl-, n-Propyl- oder eine n-Butylgruppe bzw. eine iso-Propyl-, iso- oder tert.-Butylgruppe in Betracht.
Im Falle, dass R⁶ und/oder R⁷ eine gesättigte Cycloalkylgruppe bedeutet, kommt hierfür eine Cyclopropyl-, -butyl- oder-pentylgruppe in Betracht.
R⁴ ist vorzugsweise ein Wasserstoffatom, eine Methylgruppe oder ein Chloratom.
Als Halogenatom R⁴ kommt ein Fluor-, Chlor-, Brom- oder lodatom in Frage; Chlor ist unter diesen bevorzugt.

Im Falle, daß R⁶ und/oder R⁷ eine gerad- oder verzweigtkettige Alkylgruppe mit 1 bis 4 bzw. 3 bis 4 Kohlenstoffatomen ist, kommt hierfür eine Methyl-, Ethyl-, n-Propyl- oder eine n-Butylgruppe bzw. eine iso-Propyl-, iso- oder tert.-Butylgruppe in Betracht.
R⁶ und R⁷ stehen vorzugsweise für ein Wasserstoffatom und eine Methyl-, Ethyl- oder Propylgruppe oder gemeinsam für eine Methylengruppe oder eine Doppelbindung.
Im Falle eines Alkenylrestes R⁶ und/oder R⁷ ist dies insbesondere ein Ethenylrest. Bevorzugter Vertreter für eine gesättigte Cycloalkylgruppe R⁶ und/oder R⁷ mit 3 bis 5 Kohlenstoffatomen ist der Cyclopropylrest.

Nachstehend genannte Verbindungen sind erfingdungsgemäß besonders bevorzugt:
18-Methyl-15β,16β-methylen-19-nor-20-spiroxa-4,6-dien-3-on
18-Methyl-15β,16β-methylen-19-nor-20-spirox-4-en-3-on
18-Methyl-6β,7β,15β,16β-dimethylen-19-nor-20-spirox-4-en-3-on
18-Methyl-6α,7α,15β,16β-dimethylen-19-nor-20-spirox-4-en-3-on
18-Methyl-15β,16β-methylen-7α-propyl-19-nor-20-spirox-4-en-3-on
18-Methyl-15β,16β-methylen-7β-propyl-19-nor-20-spirox-4-en-3-on
7α,18-Dimethyl-15β,16β-methylen-19-nor-20-spirox-4-en-3-on
7β,18-Dimethyl-15β,16β-methylen-19-nor-20-spirox-4-en-3-on
7α-Ethyl-18-methyl-15β,16β-methylen-19-nor-20-spirox-4-en-3-on
7β-Ethyl-18-methyl-15β,16β-methylen-19-nor-20-spirox-4-en-3-on
7α-Ethenyl-18-methyl-15β,16β-methylen-19-nor-20-spirox-4-en-3-on
7β-Ethenyl-18-methyl-15β,16β-methylen-19-nor-20-spirox-4-en-3-on
7α-Cyclopropyl-18-methyl-15β,16β-methylen-19-nor-20-spirox-4-en-3-on
7β-Cyclopropyl-18-methyl-15β,16β-methylen-19-nor-20-spirox-4-en-3-on
4,18-Dimethyl-15β,16β-methylen-19-nor-20-spirox-4-en-3-on
4-Chlor-18-methyl-15β,16β-methylen-19-nor-20-spirox-4-en-3-on
4,18-Dimethyl-6β,7β,15β,16β-dimethylen-19-nor-20-spirox-4-en-3-on
4,18-Dimethyl-6α,7α,15β,16β-dimethylen-19-nor-20-spirox-4-en-3-on
4-Chlor-18-methyl-6β,7β,15β,16β-dimethylen-19-nor-20-spirox-4-en-3-on
4-Chlor-18-methyl-6α,7α,15β,16β-dimethylen-19-nor-20-spirox-4-en-3-on.

EP-150,157 beschreibt entsprechende Verbindungen, die eine Lakton-Gruppe aufweisen.

Drospirenon (6β,7β-15β,16β-Dimethylen-3-oxo-17-pregn-4-en-21,17β-carbolacton) ist ein neues Gestagen, welches beispielsweise in dem oralen Kontrazeptivum YASMIN^{®} und dem Präparat ANGELIQ^{®} zur Behandlung postmenopausaler Beschwerden (beide SCHERING AG) enthalten ist. Aufgrund seiner vergleichsweise geringen Affinität zum Gestagenrezeptor und seiner vergleichsweise hohen Ovulationshemmdosis ist 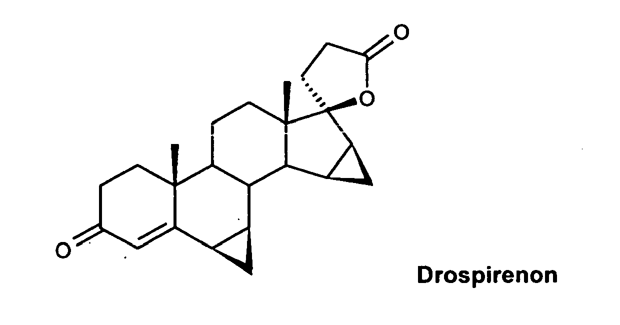 Drospirenon in YASMIN^{®} in der relativ hohen täglichen Dosis von 3 mg enthalten. Drospirenon zeichnet sich dadurch aus, daß es zusätzlich zur gestagenen Wirkung über aldosteronantagonistische (antimineralcorticoide) sowie antiandrogene Wirkung verfügt. Diese beiden Eigenschaften machen das Drospirenon in seinem pharmakologischen Profil dem natürlichen Gestagen Progesteron sehr ähnlich, welches aber anders als das Drospirenon nicht ausreichend oral bioverfügbar ist.

Aufgabe der vorliegenden Erfindung ist es deshalb, Verbindungen zur Verfügung zu stellen, die über ein weniger dissoziiertes Profil als das Drospirenon hinsichtlich ihrer Bindung an den Progesteron- und Mineralcorticoidrezeptor, vorzugsweise auch über eine stärkere Bindung als das Drospirenon an den Progesteronrezeptor verfügen. Vorzugsweise sollen die neuen Verbindungen hinsichtlich ihrer gestagenen Wirkung potenter als das Drospirenon, hinsichtlich ihrer antimineralcorticoiden Wirkung aber schwächer als das Drospirenon oder diesem vergleichbar sein.

Diese Aufgabe wird durch die Bereitstellung der hier beschriebenen 18-Methyl-15β,16β-methylen-19-nor-20-spirox-4-en-3-one der allgemeinen Formel I gelost. Die neuen Verbindungen zeichnen sich im Progesteronrezeptor-Bindungstest unter Verwendung von Cytosol aus Kaninchenuterushomogenat und von ³H-Progesteron als Bezugssubstanz durch eine vergleichbare oder höhere Affinität zum Progesteronrezeptor als Drospirenon und durch geringere Affinität zum Mineralocorticoidrezeptor aus Rattennierenhomogenat als Drospirenon aus (s. Tab. 1).

**Tabelle 1:**

| Beispiel | PR [KF] | MR [KF] | PR [RBA] | MR [RBA] |
|---|---|---|---|---|
| DRSP | 2.5 | 0.3 | 40 | 333 |
| 2 | 2.34 | 0.4 | 43 | 250 |
| 6 | 1.13 | 0.6 | 88 | 167 |
| 7 | 1.08 | 0.5 | 93 | 200 |
| 9 | 4.295 | 42 | 23 | 2 |
| 12 | 2.355 | 0.7 | 42 | 143 |
| 14 | 3.245 | 7.6 | 31 | 13 |
| 17 | 3.45 | 7.8 | 29 | 13 |
| 18 | 3.01 | 88 | 33 | 1 |
| | | | | |

| | | | | |
|---|---|---|---|---|
| PR [KF]: ³H -Progesteron = 1 ; MR [KF]: ³H -Aldosteron = 1; PR [RBA]: ³H -Progesteron = 100 ; MR [RBA]: ³H -Aldosteron = 100 Die erfindungsgemäßen Verbindungen zeichnen sich überraschenderweise durch starke gestagene Wirksamkeit aus und sind im Schwangerschaftserhaltungs-Test an der Ratte nach subcutaner Applikation stark wirksam. | | | | |

### Durchführung des Schwangerschaftserhaltungstests an der Ratte:

In graviden Ratten induziert die Entfernung der Corpora lutea oder Kastration einen Abort. Durch die exogene Zufuhr von Progestinen (Gestagenen) in Kombination mit einer geeigneten Dosis eines Estrogens, gelingt die Aufrechterhaltung der Schwangerschaft. Der Schwangerschaftserhaltungstest an ovarektomierten Ratten dient zur Bestimmung der peripheren gestagenen Aktivität einer Verbindung.

Ratten werden während des Proestrus über Nacht angepaart. Die Anpaarung wird am Morgen des darauf folgenden Tages durch die Begutachtung eines Vaginalabstriches kontrolliert. Die Anwesenheit von Spermien wird dabei als Tag 1 einer beginnenden Schwangerschaft bewertet. Am Tag 8 der Schwangerschaft werden die Tiere unter Etheranesthesie ovarektomiert. Die Behandlung mit Testverbindung und exogenem Estrogen (Estron, 5 µg/kg/Tag) wird von Tag 8 bis Tag 15 oder Tag 21 der Schwangerschaft einmal täglich subkutan ausgeführt. Die erste Anwendung am Tag 8 wird 2 Stunden vor der Kastration ausgeführt. Intakte Kontrolltiere erhalten ausschließlich Vehikel.

### Auswertung:

Am Ende des Versuches (Tag 15 oder Tag 21) werden die Tiere unter CO₂-Atmosphäre getötet und es werden lebende Föten (Föten mit schlagendem Herz) und lmplantationsstellen (frühe Resorptionen und tote Föten einschließlich Autolyse und atrophische Plazenten) in beiden uterinen Hörnern gezählt. Am Tag 22 können Föten außerdem auf Missbildungen untersucht werden. In Uteri ohne Föten oder Implantationsstellen wird die Anzahl von Nidationsstellen durch Anfärben mit 10%iger Ammoniumsulfid-Lösung ermittelt. Die Schwangerschaftserhaltungsrate wird als Quotient aus der Anzahl der lebenden Föten und der gesamten Anzahl von Nidationsstellen (sowohl resorbierte und tote Föten als auch Nidationsstellen) berechnet.

Die erfindungsgemäßen Verbindungen der allgemeinen Formel I verfügen über sehr starke gestagene Wirksamkeit bei gleichzeitiger schwacher Bindung an den Androgenrezeptor (Dissoziation).

Es wurde außerdem gefunden, daß erfindungsgemäße Verbindungen eine Kaliumretinierende, natriuretische (antimeralcorticoide) Wirkung in adrenalektomierten Ratten zeigen.

Aufgrund ihrer gestagenen Wirksamkeit können die neuen Verbindungen der allgemeinen Formel I allein oder in Kombination mit Estrogen in pharmazeutischen Präparaten zur Kontrazeption verwendet werden.
Wegen ihres günstigen Wirkungsprofils sind die erfindungsgemäßen Verbindungen besonders gut geeignet zur Behandlung prämenstrueller Beschwerden, wie Kopfschmerzen, depressive Verstimmungen, Wasserretention und Mastodynie.

Die Dosierung der erfindungsgemäßen Verbindungen in Kontrazeptionspräparaten soll 0,01 bis 5 mg, vorzugsweise 0,01 bis 2 mg pro Tag betragen.
Die Tagesdosis bei der Behandlung prämenstrueller Beschwerden liegt bei etwa 0.1 bis 20 mg.

Die gestagenen und estrogenen Wirkstoffkomponenten werden in Kontrazeptionspräparaten vorzugsweise zusammen oral appliziert. Die tägliche Dosis wird vorzugsweise einmalig verabfolgt.

Als Estrogene kommen synthetische Estrogene, vorzugsweise Ethinylestradiol, aber auch Mestranol in Betracht.

Das Estrogen wird in einer täglichen Menge verabfolgt, die der von 0.01 bis 0.04 mg Ethinylestradiol entspricht.

Die neuen Verbindungen der allgemeinen Formel I können auch in pharmazeutischen Präparaten zur Behandlung prä-, peri- und postmenopausaler Beschwerden sowie in Präparaten für die Hormon-Substitutionstherapie (HRT) eingesetzt werden.

Als Estrogene in derartigen Präparaten kommen in erster Linie natürliche Estrogene zur Anwendung, vor allem das Estradiol oder dessen Ester, beispielsweise Estradiolvalerat oder auch konjugierte Estrogene (CEEs = Conjugated Equine Estrogens), wie sie beispielsweise im Präparat PREMARIN^{®} enthalten sind.

Die Formulierung der pharmazeutischen Präparate auf Basis der neuen Verbindungen erfolgt in an sich bekannter Weise, indem man den Wirkstoff, gegebenenfalls in Kombination mit einem Estrogen, mit den in der Galenik gebräuchlichen Trägersubstanzen, Verdünnungsmitteln, gegebenenfalls Geschmackskorrigentien usw., verarbeitet und in die gewünschte Applikationsform überführt.

Für die bevorzugte orale Applikation kommen insbesondere Tabletten, Dragees, Kapseln, Pillen, Suspensionen oder Lösungen in Frage.

Für die parenterale Applikation sind insbesondere ölige Lösungen, wie zum Beispiel Lösungen in Sesamöl, Rizinusöl und Baumwollsamenöl, geeignet. Zur Erhöhung der Löslichkeit können Lösungsvermittler, wie zum Beispiel Benzylbenzoat oder Benzylalkohol, zugesetzt werden.

Es ist auch möglich, die erfindungsgemäßen Substanzen in ein transdermales System einzuarbeiten und sie damit transdermal zu applizieren.

Die neuen Verbindungen der allgemeinen Formel I werden erfingdungsgemäß wie nachstehend beschrieben hergestellt. Die Syntheseroute für die neuartigen 19-Nor-20-spiroxenone gemäß Schema 1 geht zum Beispiel von Dienolether **2** (Hofmeister et. al. Arzneim.-Forsch. 36(1), 781, 1986**)** aus.

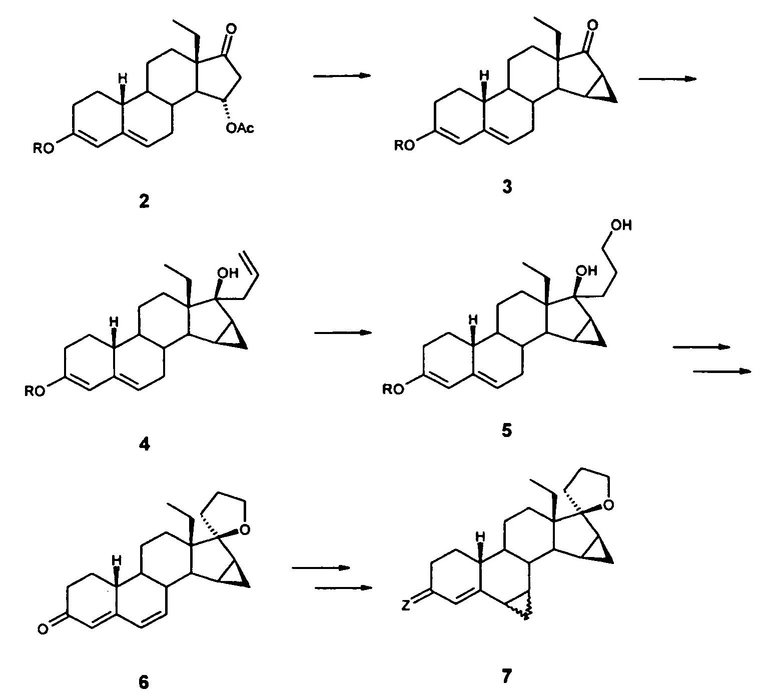

Verbindung **3** (R = Methyl) wird dann durch Methenylierung des 15-Acetates 2 nach bekannten Verfahren zum Beispiel mit Dimethylsulfoxoniummethylid und Natriumhydroxid (siehe z. B. DE-A 11 83 500, DE-A 29 22 500, EP-A 0 019 690, US-A 4,291, 029; E. J. Corey und M. Chaykovsky, J.Am.Chem.Soc. 84, 867 (1962**))** hergestellt.
Nun allyliert man in 17-Stellung zum Beispiel mit Allylmagnesiumbromid in Diethylether zu einer Verbindung **4.** Durch Hydroborierung zum Beispiel mit 9-Borabicyclo[3.3.1]nonan und oxidative Aufarbeitung zum Beispiel mit Wasserstoffperoxid erhält man den primären Alkohol **5.**
Die Einführung einer Δ⁶-Doppelbindung erfolgt über Bromierung des 3,5-Dienolethers **5** sowie anschließende Bromwasserstoffabspaltung (siehe z. B. J. Fried, J.A. Edwards, Organic Reactions in Steroid Chemistry, van Nostrand Reinhold Company 1972, S. 265-374).
Die Dienoletherbromierung kann z. B. analog der Vorschrift von J.A. Zderic, Humberto Carpio, A. Bowers und Carl Djerassi in Steroids 1, 233 (1963**)** erfolgen. Die Bromwasserstoffabspaltung gelingt durch Erhitzen der 6-Bromverbindung mit basischen Reagenzien, wie z. B. LiBr oder Li₂CO₃ in aprotischen Lösungsmitteln wie Dimethylformamid bei Temperaturen von 50-120°C oder aber indem die 6-Bromverbindungen in einem Lösungsmittel wie Collidin oder Lutidin erhitzt werden zu Verbindung **6.**
Verbindung **6** wird dann durch Methenylierung der Δ⁶-Doppelbindung nach bekannten Verfahren z.B. mit Dimethylsulfoxoniummethylid (siehe z. B. DE-A 11 83 500, DE-A 29 22 500, EP-A 0 019 690, US-A 4,291, 029; E. J. Corey und M. Chaykovsky, J.Am.Chem.Soc. 84, 867 (1962**))** in eine Verbindung **7** umgewandelt, wobei ein Gemisch der α- und β-Isomeren (Verbindungen 3a/3b) erhalten wird, das z. B. durch Chromatographie in die einzelnen Isomeren getrennt werden kann.

Die Einführung eines Substituenten R⁴ kann zum Beispiel ausgehend von einer Verbindung der Formel **6** durch Epoxidierung der Δ⁴-Doppelbindung mit Wasserstoffperoxid unter alkalischen Bedingungen und Umsetzung der entstandenen Epoxide in einem geeignetem Lösungsmittel mit Säuren der allgemeinen Formel H-R⁴ behandelt, wobei -R⁴ ein Halogenatom oder ein Pseudohalogen sein kann, oder mit katalytischen Mengen Mineralsäure umsetzt und gegebenenfalls die erhaltenen 4-Bromverbindungen der allgemeinen Formel I (wobei R⁴ = Brom) mit 2,2-Difluor-2-(fluorsulfonyl)essigsäuremethylester in Dimethylformamid in Gegenwart von Kupfer(I)iodid umsetzt.

Die Einführung einer 6-Methylengruppe kann zum Beispie! ausgehend von einem 3-Amino- 3,5-dien-Derivat durch Umsetzung mit Formalin in alkoholischer Lösung unter Bildung einer 6α-Hydroxymethylgruppe und anschließender saurer Wasserabspaltung z.B. mit Salzsäure in Dioxan/Wasser erfolgen. Die Wasserabspaltung kann aber auch in der Weise erfolgen, dass zunächst die Hydroxygruppe gegen eine bessere Fluchtgruppe ausgetauscht und dann eliminiert wird. Als Fluchtgruppen sind z. B. das Mesylat, Tosylat oder Benzoat geeignet (siehe DE-A 34 02 3291, EP-A. 0 150 157, US-A 4,584,288; K. Nickisch et al., J. Med. Chem. 34, 2464 (1991**)**).

Eine weitere Möglichkeit zur Herstellung der 6- Methylenverbindungen besteht in der direkten Umsetzung der 4(5) ungesättigten 3-Ketone mit Acetalen des Formaldehyds in Gegenwart von Natriumacetat mit z. B. Phosphoroxychlorid oder Phosphorpentachlorid in geeigneten Lösungsmitteln wie Chloroform (siehe z. B. K. Annen, H. Hofmeister, H. Laurent und R. Wiechert, Synthesis 34 (1982**)).**

Die 6-Methylenverbindungen können zur Darstellung von Verbindungen der allgemeinen Formel **I,** in denen R⁶ gleich Methyl ist und R⁶ und R⁷ gemeinsam eine zusätzliche Bindung bilden, genutzt werden.

Hierzu kann man z. B. ein von D. Burn et al. in Tetrahedron 21, 1619 (1965**)** beschriebenes Verfahren anwenden, bei dem eine Isomerisierung der Doppelbindung durch Erwärmen der 6-Methylenverbindungen in Ethanol mit 5% Palladium-Kohle-Katalysator, der entweder mit Wasserstoff oder durch Erwärmen mit einer geringen Menge Cyclohexen vorbehandelt wurde, erzielt wird. Die Isomerisierung kann auch mit einem nicht vorbehandelten Katalysator erfolgen, wenn zur Reaktionsmischung eine geringe Menge Cyclohexen zugesetzt wird. Das Auftreten geringer Anteile hydrierter Produkte kann durch Zugabe eines Überschusses an Natriumacetat verhindert werden.

Die Darstellung von 6-Methyl-4,6-dien-3-on-Derivaten kann aber auch direkt erfolgen (siehe K. Annen, H. Hofmeister, H. Laurent und R. Wiechert, Lieb. Ann. 712 (1983**)).**

Verbindungen, in denen R⁶ eine α-Methylfunktion darstellt, können aus den 6-Methylenverbindungen durch Hydrierung unter geeigneten Bedingungen dargestellt werden. Die besten Ergebnisse (selektive Hydrierung der exo-Methylenfunktion) werden durch Transfer-Hydrierung erreicht (E.A. Brande, R.P. Linstead und P.W.D. Mitchell, J. Chem.Soc. 3578 (1954**)).** Erhitzt man die 6-Methylenderivate in einem geeigneten Lösungsmittel, wie z. B. Ethanol, in Gegenwart eines Hydriddonators, wie z. B. Cyclohexen, so kommt man in sehr guten Ausbeuten zu 6α-Methylderivaten. Geringe Anteile an 6β- Methylverbindung können sauer isomerisiert werden (siehe z. B. D. Burn, D. N. Kirk und V. Petrow, Tetrahedron 1619(1965**))**.

Auch die gezielte Darstellung von 6β-Alkylverbindungen ist möglich. Hierfür werden die 4(5)-ungesättigten 3-Ketone z. B. mit Ethylenglycol, Trimethylorthoformiat in Dichlormethan in Gegenwart katalytischer Mengen einer Säure, (z. B. p-Toluolsulfonsäure) zu den entsprechenden 3-Ketalen umgesetzt. Während dieser Ketalisierung isomerisiert die Doppelbindung in die Position 5(6). Eine selektive Epoxidierung dieser 5(6)-Doppelbindung gelingt z. B. durch Verwendung organischer Persäuren, z. B. m-Chlorperbenzoesäure, in geeigneten Lösungsmittel wie Dichlormethan. Alternativ hierzu kann die Epoxidierung auch mit Wasserstoffperoxid in Gegenwart von z. B. Hexachloraceton oder 3- Nitrotrifluoracetophenon erfolgen. Die gebildeten 5 α,6α-Epoxide können dann unter Verwendung entsprechender Alkylmagnesiumhalogenide oder Alkyllithiumverbindungen axial geöffnet werden. Man gelangt so zu 5α- Hydroxy-6β-Alkylverbindungen; Die Spaltung der 3-Ketoschutzgruppe kann unter Erhalt der 5α-Hydroxyfunktion durch Behandeln unter milden sauren Bedingungen (Essigsäure oder 4 n Salzsäure bei 0°C) erfolgen. Basische Eliminierung der 5α-Hydroxyfunktion mit z. B. verd. wäßriger Natronlauge ergibt die 3-Keto-4-en-Verbindungen mit einer β-ständigen 6-Alkylgruppe. Alternativ hierzu ergibt die Ketalspaltung unter drastischeren Bedingungen (wäßrige Salzsäure oder eine andere starke Säure) die entsprechenden 6α-Alkylverbindungen.

In Position 7 mit einer Alkyl-, Alkenyl- oder Cycloalkylgruppe substituierte Verbindungen können wie in den Beispielen beschrieben oder analog zu diesen Vorschriften unter Verwendung analoger zu den dort beschriebenen Reagenzien erhalten werden.

Die erhaltenen Verbindungen der allgemeinen Formel I, in denen Z für ein Sauerstoffatom steht, können gewünschten falls durch Umsetzung mit Hydroxylaminhydrochlorid in Gegenwart eines tertiären Amins bei Temperaturen zwischen -20 und +40°C in ihre entprechenden Oxime überführt werden (allgemeine Formel I mit Z in der Bedeutung von =NOH, wobei die Hydroxygruppe syn- oder antiständig sein kann). Geeignete tertiäre Basen sind beispielsweise Trimethylamin, Triäthylamin, Pyridin, N,N-Dimethylaminopyridin, 1,5- Diazabicyclo[4.3.0]non-5-en (DBN) und 1,5- Diazabicyclo[5.4.0]undec-5-en (DBU), wobei Pyridin bevorzugt ist. Dies geht analog wie es in WO 98/24801 für die Herstellung entsprechender 3-Oxyimino-Derivate des Drospirenons beschrieben ist.

Die Entfernung der 3-Oxogruppe zur Herstellung eines Endprodukts der allgemeinen Formel I mit Z in der Bedeutung von zwei Wasserstoffatomen kann beispielsweise nach der in DE-A 28 05 490 angegebenen Vorschrift durch reduktive Spaltung eines Thioketals der 3- Ketoverbindung erfolgen.

Die nachfolgenden Beispiele dienen der näheren Erläuterung der Erfindung:

### Beispiel 1

### 18-Methyl-15β,16β-methylen-19-nor-20-spiroxa-4,6-dien-3-on

### a) 3-Methoxy-18-methyl-15β,16β-methylen-estra-3,5-dien-17-on

Eine Suspension von 92.0 g 15α-Acetoxy-3-methoxy-18-methyl-estra-3,5-dien-17-on (Hofmeister et. al. Arzneim.-Forsch. 36(1), 781, 1986) in 500 ml Dimethylsulfoxid gab man in eine Suspension von 254 g Trimethylsulfoxoniumiodid in 1165 ml Dimethylsulfoxid, die zuvor 2 Stunden mit 43,5 g Natriumhydroxid bei Raumtemperatur unter Argon gerührt wurde. Dieses wurde weitere 1.5 Stunden bei Raumtemperatur gerührt. Anschließend rührte man in 15 I Eiswasser/Kochsalz ein, filtrierte den Niederschlag ab, wusch mit Wasser, trocknete im Vakuum bei 60 °C. Man erhielt 94.5 g 3-Methoxy-18-methyl-15β,16β-methylen-estra-3,5-dien-17-on als Rohprodukt. 134-135 °C, [α]_{D} -215,2 ° (Chloroform, c = 9.9 mg/ml)

### b) 3-Methoxy-18-methyl-15β,16β-methylen-17α-(prop-2-enyl)-estra-3,5-dien-17β-ol

Eine Lösung von 75.8 g 3-Methoxy-18-methyl-15β,16β-methylen-estra-3,5-dien-17-on in 920 ml Dichlormethan wurde bei 0 °C mit 574 ml einer 1 M Lösung Allylmagnesiumbromid in Diethylether versetzt, und 1 Stunde unter Argon bei 0 °C gerührt. Anschließend tropfte man 290 ml einer gesättigten Ammoniumchloridlösung bei 0 °C zu, rührte 0.5 Stunden bei 0 °C, gab auf Wasser, nahm in Ethylacetat auf, wusch mit Wasser neutral, trocknete über Natriumsulfat, und engte im Vakuum ein. Man erhielt 86.5 g 3-Methoxy-18-methyl-15β,16β-methylen-17α-(prop-2-enyl)-estra-3,5-dien-17β-ol als Rohprodukt. Kristalle der reinen Verbindung hatten einen Schmelzpunkt von 110-112 °C, [α]_{D} = -80.2 ° (Chloroform, c = 9.94 mg/ml).

### c) 17α-(3-Hydroxypropanyl)-3-methoxy-18-methyl-15β,16β-methylen-estra-3,5-dien-17β-ol

Eine Lösung von 86.5 g 3-Methoxy-18-methyl-15β,16β-methylen-17α-(prop-2-enyl)-estra-3,5-dien-17β-ol in 1 I Tetrahydrofuran wurde bei 25 °C mit 1.5 einer 0.5 M 9-Borabicylo[3.3.1]nonan-Lösung in Tetrahydrofuran versetzt, und 4 Stunden unter Argon bei 25 °C gerührt, anschließend wurden bei 0 °C 33.77 g Natriumhydroxid in 475 ml Wasser langsam zugetropft, 5 Minuten bei 25 °C gerührt, 172 ml 30%iges Wasserstoffperoxid langsam zugetropft, und 18 Stunden bei 25 °C gerührt. Anschließend rührte man in Eiswasser/Kochsalz ein, filtrierte den Niederschlag ab, wusch mit Wasser, trocknete im Vakuum bei 60 °C zur Trockne ein. Man erhielt 91.5 g 17α-(3-Hydroxypropanyl)-3-methoxy-18-methyl-15β,16β-methylen-estra-3,5-dien-17β-ol als Rohprodukt. Kristalle der reinen Verbindung hatten einen Schmelzpunkt von 152-154 °C, [α]_{D} = -155.2 ° (Chloroform, c = 9.76 mg/ml).

### d) 17β-Hydroxy-17α-(3-hydroxypropanyl)-18-methyl-15β,16β-methylen-estra-4,6-dien-3-on

Eine Suspension von 91.5 g 17α-(3-Hydroxypropanyl)-3-methoxy-18-methyl-15β,16β-methylen-estra-3,5-dien-17β-ol in 915 ml 1-Methyl-2-pyrrolidon wurde nacheinander bei 0 °C mit 91.5 ml einer 10%igen Natrumacetatlösung sowie bei dieser Temperatur mit 35.9 g 1,3-Dibrom-5,5-dimethylhydantoin portionsweise versetzt, 0.5 Stunden bei 0°C (Eisbad) gerührt, mit 34 g Lithiumbromid sowie 29.9 g Lithiumcarbonat versetzt, und 3.5 Stunden bei 100°C Badtemperatur gerührt. Anschließend rührte man in Eiswasser /Kochsalz ein, filtrierte den Niederschlag ab, wusch mit Wasser, und rührte im feuchten Zustand mit 250 ml Ethylacetat aus. Man erhielt 44.1 g 17β-Hydroxy-17α-(3-hydroxypropanyl)-18-methyl-15β,16β-methylen-estra-4,6-dien-3-on als Kristalle vom Schmelzpunkt 132-135 °C, [α]_{D} = -14.0 **°** (Pyridin**,** c = 4.28 mg/ml).

### e) 18-Methyl-15β,16β-methylen-19-nor-20-spiroxa-4,6-dien-3-on

Eine Lösung von 1.07 g 17β-Hydroxy-17α-(3-hydroxypropanyl)-18-methyl-15β,16β-methylen-estra-4,6-dien-3-on in 2.5 ml Pyridin wird mit 720 mg p-Toluolsulfonsäurechlorid versetzt und 18 Stunden bei Raumtemperatur gerührt. Anschließend wurde auf Wasser gegeben, dreimal mit Ethylacetat extrahiert, die vereinigten organischen Phasen mit 1 M Salzsäure, Wasser und Kochsalzlösung neutralgewaschen, über Natriumsulfat getrocknet, im Vakuum eingeengt, und an Kieselgel mit Hexan / Essigester chromatographiert. Man erhielt 630 mg reines 18-Methyl-15β,16β-methylen-19-nor-20-spiroxa-4,6-dien-3-on. Nach Kristallisation aus Aceton/Hexan erhielt man Kristalle vom Schmelzpunkt 134-135 °C, [α]_{D} = -80.6 ° (Chloroform, c = 10.03 mg/ml).

### Beispiel 2

### 18-Methyl-6α,7α,15β,16β-dimethylen-19-nor-20-spirox-4-en-3-on

Eine Suspension von 9.41 g Trimethylsulfoxoniumiodid in 210 ml Dimethylsulfoxid wurde mit 1.71 g Natriumhydrid (60% in Öl) 2 Stunden bei Raumtemperatur unter Argon gerührt, mit 5.7 g 18-Methyl-15β,16β-methylen-19-nor-20-spiroxa-4,6-dien-3-on (Beispiel 1) versetzt und 20 Stunden bei Raumtemperatur gerührt. Zur Aufarbeitung wurde auf Wasser gegeben, dreimal mit Ethylacetat extrahiert, mit Wasser und Kochsalzlösung neutralgewaschen, über Natriumsulfat getrocknet, im Vakuum zur Trockne eingeengt und an Kieselgel mit Dichlormethan / Aceton chromatographiert. Man erhielt als Fraktion II der Chromatographie 438 mg 18-Methyl-6α,7α,15β,16β-dimethylen-19-nor-20-spirox-4-en-3-on. Nach Kristallisation aus Aceton erhielt man Kristalle vom Schmelzpunkt 228-230 °C, [α]_{D} = +40.2° +/- 0.2° (Chloroform, c = 11.1 mg/ml)

### Beispiel 3

### 18-Methyl-6β,7β,15β,16β-dimethylen-19-nor-20-spirox-4-en-3-on

Nach der Methode des Beispiels 2 erhielt man als Fraktion 1 der Chromatographie 1.2 g 18-Methyl-6β,7β,15β,16β-dimethylen-19-nor-20-spirox-4-en-3-on. Nach Kristallisation aus Aceton/Hexan erhielt man Kristalle vom Schmelzpunkt 154-155 °C, [α]_{D} = -175.1 ° (Chloroform, c = 9.5 mg/ml)

### Beispiel 4

### 18-Methyl-15β,16β-methylen-7α-propyl-19-nor-20-spirox-4-en-3-on

In eine Lösung von 1.0 g 18-Methyl-15β,16β-methylen-19-nor-20-spiroxa-4,6-dien-3-on (Beispiel 1) in 20 ml Tetrahydrofuran gab man bei Raumtemperatur 31.2 mg Kupfer-I-chlorid und rührte 10 Minuten bevor man auf-15°C abkühlte, mit 200 mg Aluminiumchlorid versetzte, 30 Minuten bei dieser Temperatur rührte, mit 3.34 ml Propylmagnesiumbromidlösung (2 M in Tetrahydrofuran) tropfenweise versetzte, und eine Stunde bei -15 °C rührte. Zur Aufarbeitung wurde die Reaktionsmischung mit bei - 15 °C mit 3 ml 2 M Salzsäure versetzt, 0.5 Stunden bei Raumtemperatur gerührt, auf Wasser gegeben, dreimal mit Essigester extrahiert, über Natriumsulfat getrocknet, im Vakuum eingeengt, und an Kieselgel mit Hexan / Ethylacetat chromatographiert. Nach Kristallisation der Fraktion I erhielt man 233 mg 18-Methyl-15β,16β-methylen-7α-propyl-19-nor-20-spirox-4-en-3-on als Kristalle vom Schmelzpunkt 142-143 °C, [α]_{D} = -2.7 ° (Chloroform, c = 9.5 mg/ml)

### Beispiel 5

### 18-Methyl-15β,16β-methylen-7β-propyl-19-nor-20-spirox-4-en-3-on

Nach der Methode des Beispiels 4 erhielt man nach der Chromatographie als Fraktion II 241 mg 18-Methyl-15β,16β-methylen-7β-propyl-19-nor-20-spirox-4-en-3-on als Feststoff vom Schmelzpunkt 87-88 °C, [α]_{D} = -10.8 ° (Chloroform, c = 10.0 mg/ml).

### Beispiel 6

### 7α,18-Dimethyl-15β,16β-methylen-19-nor-20-spirox-4-en-3-on

Nach der Methode des Beispiels 4 mit 3 M-Methylmagnesiumbromid in Ether anstelle des Propylmagnesiumbromids erhielt man nach der Chromatographie als Fraktion I 483 mg 7α,18-Dimethyl-15β,16β-methylen-19-nor-20-spirox-4-en-3-on als Feststoff vom Schmelzpunkt 190-191 °C, [α]_{D} = 6.5 **°** (Chloroform, c = 10.16 mg/ml).

### Beispiel 7

### 7β,18-Dimethyl-15β,16β-methylen-19-nor-20-spirox-4-en-3-on

Nach der Methode des Beispiels 6 erhielt man nach der Chromatographie als Fraktion II 201 mg 7β,18-Dimethyl-15β,16β-methylen-19-nor-20-spirox-4-en-3-on als Feststoff vom Schmelzpunkt 172-173 °C, [a]_{D} = -11.2 ° (Chloroform, c = 10.35 mg/ml).

### Beispiel 8

### 7α-Ethyl-18-methyl-15β,16β-methylen-19-nor-20-spirox-4-en-3-on

Nach der Methode des Beispiels 4 mit 3 M-Ethylmagnesiumbromid in Ether anstelle des Propylmagnesiumbromids erhielt man nach der Chromatographie als Fraktion I 453 mg 7α-Ethyl-18-methyl-15β,16β-methylen-19-nor-20-spirox-4-en-3-on als Feststoff vom Schmelzpunkt 197-198 °C, [α]_{D} = -6.7 ° (Chloroform, c = 10.42 mg/ml).

### Beispiel 9

### 7β-Ethyl-18-methyl-15β,16β-methylen-19-nor-20-spirox-4-en-3-on

Nach der Methode des Beispiels 8 erhielt man nach der Chromatographie als Fraktion II 113 mg 7β-Ethyl-18-methyl-15β,16β-methylen-19-nor-20-spirox-4-en-3-on als Feststoff vom Schmelzpunkt 185-187 °C, [α]_{D} = -11.7 ° (Chloroform, c = 9.4 mg/ml).

### Beispiel 10

### 7α-Ethenyl-18-methyl-15β,16β-methylen-19-nor-20-spirox-4-en-3-on

Nach der Methode des Beispiels 4 erhielt man nach der Chromatographie als Fraktion I 280.6 mg 7α-Ethenyl-18-methyl-15β,16β-methylen-19-nor-20-spirox-4-en-3-on als Feststoff vom Schmelzpunkt 188-190 °C, [a]_{D} = -59.8 ° (Chloroform, c = 9.87 mg/ml).

### Beispiel 11

**7**β**-Ethenyl-18-methyl-15**β**,16**β**-methylen-19-nor-20-spirox-4-en-3-on**Nach der Methode des Beispiels 4 erhielt man nach der Chromatographie als Fraktion II 54.4 mg 7β-Ethenyl-18-methyl-15β,16β-methylen-19-nor-20-spirox-4-en-3-on als Feststoff vom Schmelzpunkt 149-150 °C. [α]_{D} = -37.6 ° (Chloroform, c = 5.11 mg/ml).

### Beispiel 12

### 7α-Cyclopropyl-18-methyl-15β,16β-methylen-19-nor-20-spirox-4-en-3-on

Nach der Methode des Beispiels 4 erhielt man nach der Chromatographie als Fraktion I 360 mg 7α-Cyclopropyl-18-methyl-15β,16β-methylen-19-nor-20-spirox-4-en-3-on als Feststoff vom Schmelzpunkt 167-168 °C. [a]_{D} = -55.3 ° (Chloroform, c = 10.14 mg/ml).

### Beispiel 13

### 7β-Cyclopropyl-18-methyl-15β,16β-methylen-19-nor-20-spirox-4-en-3-on

Nach der Methode des Beispiels 4 erhielt man nach der Chromatographie als Fraktion II 63 mg 7β-Cyclopropyl-18-methyl-15β,16β-methylen-19-nor-20-spirox-4-en-3-on als Feststoff vom Schmelzpunkt 124-126 °C. [a]_{D} = -16.9 ° (Chloroform, c = 10.18 mg/ml).

### Beispiel 14

### 4,18-Dimethyl-15β,16β-methylen-19-nor-20-spirox-4-en-3-on

### a) 15α-Acetoxy-3,3-ethylendioxy-18-methyl-19-nor-androst-5-en-17-on

Eine Lösung von 10 g 15α-Acetoxy-3-methoxy-18-methyl-estra-3,5-dien-17-on in 140 ml Dichlormethan wurde mit 40 ml Ethylenglykol sowie 27.5 ml Trimethylorthoformiat versetzt und nach Zugabe von 670 mg para-Toluolsulfonsäure 1 Stunde bei Raumtemperatur gerührt. Anschließend wurde mit 1.85 ml Pyridin versetzt mit Dichlormethan verdünnt, mit gesättigter Natriumhydrogencarbonatlösung, Wasser und Kochsalzlösung gewaschen, über Natriumsulfat getrocknet und im Vakuum eingeengt. Man erhielt 11.1 g rohes 15α-Acetoxy-3,3-ethylendioxy-18-methyl-19-nor-androst-5-en-17-on.

### b) 3,3-Ethylendioxy-18-methyl-15β,16β-methylen-19-nor-androst-5-en-17-on

Eine Suspension von 28.75 g Trimethylsulfoxoniumiodid in 210 ml Dimethylsulfoxid wurde mit 4.92 g Natriumhydrid (60% in Öl) 2 Stunden bei Raumtemperatur unter Argon gerührt, mit 11.1 g 15α-Acetoxy-3,3-ethylendioxy-18-methyl-19-nor-androst-5-en-17-on versetzt und 20 Stunden bei Raumtemperatur gerührt. Zur Aufarbeitung wurde auf Wasser gegeben, dreimal mit Ethylacetat extrahiert, mit Wasser und Kochsalzlösung neutralgewaschen, über Natriumsulfat getrocknet und im Vakuum zur Trockne eingeengt. Man erhielt 10.2 g rohes 3,3-Ethylendioxy-18-methyl-15β,16β-methylen-19-nor-androst-5-en-17-on Nach Kristallisation aus Aceton erhielt man Kristalle vom Schmelzpunkt 221,7 °C.

### c) 3,3-Ethylendioxy-18-methyl-15β,16β-methylen-17α-(prop-2-enyl)-19-nor-androst-5-en-17β-ol

Eine Lösung von 10.2 g 3,3-Ethylendioxy-18-methyl-15β,16β-methylen-19-nor-androst-5-en-17-on in 120 ml Dichlormethan wurde bei 0 °C langsam mit 71 ml einer 1 M-Allylmagnesiumbromidlösung in Diethylether versetzt und eine Stunde bei 0 °C gerührt. Anschließend wurde tropfenweise mit 40 ml einer gesättigten Ammoniumchloridlösung versetzt, 0.5 Stunden bei 0 °C gerührt, auf Wasser gegeben, mit Ethylacetat extrahiert, mit Wasser und Kochsalzlösung neutralgewaschen, über Natriumsulfat getrocknet und im Vakuum zur Trockne eingeengt. Nach Chromatographie an Kieselgel mit Hexan /Ethylacetat erhielt man 7.33 g reines 3,3-Ethylendioxy-18-methyl-15β,16β-methylen-17α-(prop-2-enyl)-19-nor-androst-5-en-17β-ol.

### d) 3,3-Ethylendioxy-17α-(3-hydroxypropyl)-18-methyl-15β,16β-methylen-19-nor-androst-5-en-17β-ol

Eine Lösung von 80.3 g 3,3-Ethylendioxy-18-methyl-15β,16β-methylen-17α-(prop-2-enyl)-19-nor-androst-5-en-17β-ol in 900 ml Tetrahydrofuran wurde bei 25 °C mit 1.4 I einer 0.5 M 9-Borablcyclo[3.3.1]nonan-Lösung in Tetrahydrofuran versetzt, und 4 Stunden unter Argon bei 25 °C gerührt, anschließend wurden 30.5 g Natriumhydroxid in 425 ml Wasser bei 0 °C langsam zugetropft, 5 Minuten bei 25 °C gerührt, 155 ml 30%iges Wasserstoffperoxid langsam zugetropft, und 18 Stunden bei 25 °C gerührt. Anschließend verdünnte man mit Ethylacetat, wusch mit Wasser, trocknete über Natriumsulfat und engte im Vakuum bei 60 °C zur Trockne ein. Man erhielt 80.7 g 3,3-Ethylendioxy-17α-(3-hydroxypropyl)-18-methyl-15β,16β-methylen-19-nor-androst-5-en-17β-ol als Öl.

### e) 3,3-Ethylendioxy-18-methyl-15β,16β-methylen-19-nor-20-spirox-5-en

Eine Lösung von 80.7 g 3,3-Ethylendioxy-17α-(3-hydroxypropyl)-18-methyl-15β,16β-methylen-19-nor-androst-5-en-17β-ol in 170 ml Pyridin wurde mit 48 g para-Toluolsulfonsäurechlorid versetzt und 24 Stunden bei 25 °C gerührt. Dann verdünnte man mit Ethylacetat, wusch mit Wasser sowie gesättigter Kochsalzlösung neutral, trocknete über Natriumsulfat und engte im Vakuum bei 60 °C zur Trockne ein. Man erhielt 75.8 g Rohprodukt. Nach Chromatographie an Kieselgel mit Hexan / Essigester erhielt man 50.5 g reines 3,3-Ethylendioxy-18-methyl-15β,16β-methylen-19-nor-20-spirox-5-en. Kristalle der reinen Verbindung hatten einen Schmelzpunkt von 58-60 °C, [α]_{D} = -9.3 ° (Chloroform, c = 10.59 mg/ml).

### f) 18-Methyl-15β,16β-methylen-19-nor-20-spirox-4-en-3-on

Eine Lösung von 50.5 g 3,3-Ethylendioxy-18-methyl-15β,16β-methylen-19-nor-20-spirox-5-en in 500 ml Methanol wurde mit 50 ml wässriger Schwefelsäure (8%ig) versetzt und 8.5 Stunden bei 25 °C gerührt. Anschließend gab man auf Natriumhydrogencarbonatlösung, extrahierte dreimal mit Ethylacetat, wusch mit Wasser neutral, trocknete über Natriumsulfat und engte im Vakuum bei 50 °C zur Trockne ein. Man erhielt 46.2 g Rohprodukt. Nach Chromatographie an Kieselgel mit Dichlormethan /Aceton erhielt man 25,8 g reines 18-Methyl-15β,16β-methylen-19-nor-20-spirox-4-en-3-on. Kristalle der reinen Verbindung hatten einen Schmelzpunkt von 208-210 °C, [α]_{D} = +4.4 ° (Chloroform, c = 10.1 mg/ml).

### g) 4,18-Dimethyl-15β,16β-methylen-19-nor-20-spirox-4-en-3-on Zu einer Lösung von 508 mg Kalium-tert-butylat in 20 ml tert-Butanol gab man bei 100

°C Badtemperatur eine Lösung von 1 g 18-Methyl-15β,16β-methylen-19-nor-20-spirox-4-en-3-on in 20 ml tert-Butanol sowie über 4 Stunden eine zweite Lösung von 1.46 ml lodmethan in 50 ml tert-Butanol und rührte noch eine Stunde bei 100 °C Badtemperatur. Dann engte man im Vakuum auf ein drittel des Volumens ein, verdünnte man mit Ethylacetat, wusch zweimal mit Wasser sowie dreimal mit gesättigter Kochsalzlösung, trocknete über Natriumsulfat und engte im Vakuum zur Trockne ein. Man erhielt 1.1 g Rohprodukt. Nach Chromatographie an Kieselgel mit Hexan / Ethylacetat erhielt man 301.2 mg 4,18-Dimethyl-15β,16β-methylen-19-nor-20-spirox-4-en-3-on als Feststoff vom Schmelzpunkt 155-156 °C. [α]_{D} = +1 ° (Chloroform, c = 10.75 mg/ml).

### Beispiel 15

### 4-Chlor-18-methyl-15β,16β-methylen-19-nor-20-spirox-4-en-3-on

Zu einer Lösung von 1 g 18-Methyl-15β,16β-methylen-19-nor-20-spirox-4-en-3-on in 10 ml Pyridin gab man bei 0 °C Badtemperatur 0.38 ml Sulfurylchlorid und rührte 3 Stunden weiter. Dann gab man auf Wasser, extrahierte dreimal mit Ethylacetat, wusch mit Wasser neutral, trocknete über Natriumsulfat und engte im Vakuum zur Trockne ein. Man erhielt 1.2 g Rohprodukt. Nach Chromatographie an Kieselgel mit Hexan /Ethylacetat erhielt man 604.8 mg reines 4-Chlor-18-methyl-15β,16β-methylen-19-nor-20-spirox-4-en-3-on als Feststoff vom Schmelzpunkt 149-151 °C. [α]_{D} = +9.4 ° (Chloroform, c = 11.06 mg/ml).

### Beispiel 16

### 4,18-Dimethyl-6α,7α,15β,16β-dimethylen-19-nor-20-spirox-4-en-3-on

Nach der Methode des Beispiels 14 erhielt man aus 0.5 g 18-Methyl-6α,7α,15β,16β-dimethylen-19-nor-20-spirox-4-en-3-on 220 mg 4,18-Dimethyl-6α,7α,15β,16β-dimethylen-19-nor-20-spirox-4-en-3-on als Feststoff vom Schmelzpunkt 190-191 °C. [α]_{D} = +103,3 ° (Chloroform, c = 10.22 mg/ml).

### Beispiel 17

### 4,18-Dimethyl-6β,7β,15β,16β-dimethylen-19-nor-20-spirox-4-en-3-on

Nach der Methode des Beispiels 14 erhielt man aus 0.66 g 18-Methyl-6β,7β,15β,16β-dimethylen-19-nor-20-spirox-4-en-3-on 186.7 mg 4,18-Dimethyl-6β,7β,15β,16β-dimethylen-19-nor-20-spirox-4-en-3-on als Feststoff vom Schmelzpunkt 175-177 °C. [α]_{D} = -230.7 ° (Chloroform, c = 10.79 mg/ml).

### Beispiel 18

### 4-Chlor-18-methyl-6β,7β,15β,16β-dimethylen-19-nor-20-spirox-4-en-3-on

Nach der Methode des Beispiels 15 erhielt man aus 0.66 g 18-Methyl-6β,7β,15β,16β-dimethylen-19-nor-20-spirox-4-en-3-on 303.4 mg 4-Chlor-18-methyl-6β,7β,15β,16β-dimethylen-19-nor-20-spirox-4-en-3-on als Feststoff vom Schmelzpunkt 152-153 °C. [α]_{D} = -222.7 ° (Chloroform, c = 10.30 mg/ml).

### Beispiel 19

### 4-Chlor-18-methyl-6α,7α,15β,16β-dimethylen-19-nor-20-spirox-4-en-3-on

Nach der Methode des Beispiels 15 erhielt man aus 534 mg 18-Methyl-6α,7α,15β,16β-dimethylen-19-nor-20-spirox-4-en-3-on 128 mg 4-Chlor-18-methyl-6α,7α,15β,16β-dimethylen-19-nor-20-spirox-4-en-3-on als Feststoff vom Schmelzpunkt 177-178 °C. [α]_{D} = +80.0 ° (Chloroform, c = 9.94 mg/ml).

## Patentansprüche

1. 18-Methyl-15β,16β-methylen-19-nor-20-spirox-4-en-3-one der allgemeinen Formel I 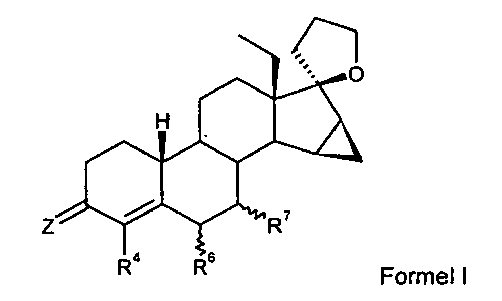 worin
Z ein Sauerstoffatom, zwei Wasserstoffatome, eine Gruppierung =NOR oder
=NNHSO₂R, wobei R ein Wasserstoffatom oder eine gerad- oder verzweigtkettige Alkylgruppe mit 1 bis 4 bzw. 3 bis 4 Kohlenstoffatomen ist,
R⁴ ein Wasserstoffatom, ein Halogenatom oder eine Trifluormethylgruppe,
und R⁶ und/oder R⁷ α- oder β-ständig sein kann und R⁶ und R⁷ unabhängig voneinander ein Wasserstoffatom oder eine gerad- oder verzweigtkettige Alkylgruppe mit 1 bis 4 bzw. 3 bis 4 Kohlenstoffatomen oder eine gerad- oder verzweigtkettige Alkenylgruppe mit 2 bis 4 bzw. 3 bis 4 Kohlenstoffatomen oder eine gesättigte Cycloalkylgruppe mit 3 bis 5 Kohlenstoffatomen oder gemeinsam eine Methylengruppe oder eine Doppelbindung,
bedeuten.

2. 18-Methyl-15β,16β-methylen-19-nor-20-spirox-4-en-3-one nach Anspruch 1 der allgemeinen Formel I, **dadurch gekennzeichnet, dass** Z ein Sauerstoffatom ist.

3. 18-Methyl-15β,16β-methylen-19-nor-20-spirox-4-en-3-one nach Anspruch 1 der allgemeinen Formel I, **dadurch gekennzeichnet, dass** R⁴ ein Wasserstoffatom, eine Methylgruppe oder ein Chloratom ist.

4. 18-Methyl-15β,16β-methylen-19-nor-20-spirox-4-en-3-one nach Anspruch 1 der allgeminen Formel I, **dadurch gekennzeichnet, dass** R⁶ und R⁷ gemeinsam eine 6α,7α- oder 6β,7β-Methylengruppe oder eine zusätzliche Bindung bedeuten.

5. 18-Methyl-15β, 16β-methylen-19-nor-20-spirox-4-en-3-one nach Anspruch 1 der allgemeinen Formel I, **dadurch gekennzeichnet, dass** R⁷ eine α- oder β-ständige Methyl-, Ethyl-, Propyl- oder Ethenylgruppe oder einen α- oder β-ständigen Cyclopropylrest bedeutet.

6. Verbindungen nach Anspruch 1, nämlich
18-Methyl-15β,16β-methylen-19-nor-20-spiroxa-4,6-dien-3-on 18-Methyl-15β,16β-methylen-19-nor-20-spirox-4-en-3-on 18-Methyl-6β,7β,15β,16β-dimethylen-19-nor-20-spirox-4-en-3-on 18-Methyl-6α,7α,15β,16β-dimethylen-19-nor-20-spirox-4-en-3-on 18-Methyl-15β,16β-methylen-7α-propyl-19-nor-20-spirox-4-en-3-on 18-Methyl-15β,16β-methylen-7β-propyl-19-nor-20-spirox-4-en-3-on 7α,18-Dimethyl-15β,16β-methylen-19-nor-20-spirox-4-en-3-on 7β,18-Dimethyl-15β,16β-methylen-19-nor-20-spirox-4-en-3-on 7α-Ethyl-18-methyl-15β,16β-methylen-19-nor-20-spirox-4-en-3-on 7β-Ethyl-18-methyl-15β,16β-methylen-19-nor-20-spirox-4-en-3-on 7α-Ethenyl-18-methyl-15β,16β-methylen-19-nor-20-spirox-4-en-3-on 7β-Ethenyl-18-methyl-15β,16β-methylen-19-nor-20-spirox-4-en-3-on 7α-Cyclopropyl-18-methyl-15β,16β-methylen-19-nor-20-spirox-4-en-3-on 7β-Cyclopropyl-18-methyl-15β,16β-methylen-19-nor-20-spirox-4-en-3-on 4,18-Dimethyl-15β,16β-methylen-19-nor-20-spirox-4-en-3-on 4-Chlor-18-methyl-15β,16β-methylen-19-nor-20-spirox-4-en-3-on 4,18-Dimethyl-6β,7β,15β,16β-dimethylen-19-nor-20-spirox-4-en-3-on 4,18-Dimethyl-6α,7α,15β,16β-dimethylen-19-nor-20-spirox-4-en-3-on 4-Chlor-18-methyl-6β,7β,15β,16β-dimethylen-19-nor-20-spirox-4-en-3-on 4-Chlor-18-methyl-6α,7α,15β,16β-dimethylen-19-nor-20-spirox-4-en-3-on.

7. Pharmazeutische Präparate enthaltend mindestens eine Verbindung gemäß einem der Ansprüche 1 bis 6 sowie einen pharmazeutisch unbedenklichen Träger.

8. Pharmazeutische Präparate nach Anspruch 7, außerdem enthaltend mindestens ein Estrogen.

9. Pharmazeutische Präparate nach Anspruch 8 enthaltend Ethinylestradiol.

10. Pharmazeutische Präparate nach Anspruch 8, enthaltend ein natürliches Estrogen.

11. Pharmazeutische Präparate nach Anspruch 10, enthaltend Estradiol.

12. Pharmazeutische Präparate nach Anspruch 10, enthaltend Estradiolvalerat.

13. Pharmazeutische Präparate nach Anspruch 10, enthaltend mindestens ein konjugiertes Estrogen.

## Claims

1. 18-Methyl-15β,16β-methylene-19-nor-20-spirox-4-en-3-ones of the general formula I 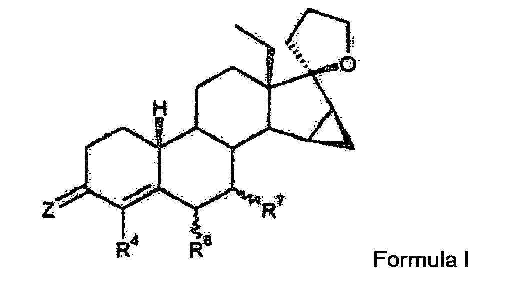 in which
Z is an oxygen atom, two hydrogen atoms, a group =NOR or =NNHSO2R, where R is a hydrogen atom or a straight- or branched-chain alkyl group having 1 to 4 or 3 to 4 carbon atoms,
R⁴ is a hydrogen atom, a halogen atom or a trifluoromethyl group,
and R⁶ and/or R⁷ may have α or β configuration, and R⁶ and R⁷ are independently of one another a hydrogen atom or a straight- or branched-chain alkyl group having 1 to 4 or 3 to 4 carbon atoms or a straight- or branched-chain alkenyl group having 2 to 4 or 3 to 4 carbon atoms or a saturated cycloalkyl group having 3 to 5 carbon atoms or together are a methylene group or a double bond.

2. 18-Methyl-15β,16β-methylene-19-nor-20-spirox-4-en-3-ones according to Claim 1 of the general formula I, **characterized in that** Z is an oxygen atom.

3. 18-Methyl-15β,16β-methylene-19-nor-20-spirox-4-en-3-ones according to Claim 1 of the general formula I, **characterized in that** R⁴ is a hydrogen atom, methyl group or chlorine atom.

4. 18-Methyl-15β,16β-methylene-19-nor-20-spirox-4-en-3-ones according to Claim 1 of the general formula I, **characterized in that** R⁶ and R⁷ together are a 6α,7α- or 6β,7β-methylene group or an additional bond.

5. 18-Methyl-15β,16β-methylene-19-nor-20-spirox-4-en-3-ones according to Claim 1 of the general formula I, **characterized in that** R⁷ is an α- or β-configured methyl, ethyl, propyl or ethenyl group or an α- or β- configured cyclopropyl radical.

6. Compounds according to Claim 1, specifically 18-methyl-15β,16β-methylene-19-nor-20-spiroxa-4,6-dien-3-one 18-methyl-15β,16β-methylene-19-nor-20-spirox-4-en-3-one 18-methyl-6β,7β,15β,16β-dimethylene-19-nor-20-spirox-4-en-3-one 18-methyl-6α,7α,15β,16β-dimethylene-19-nor-20-spirox-4-en-3-one 18-methyl-15β,16β-methylene-7a-propyl-19-nor-20-spirox-4-en-3-one 18-methyl-15β,16β-methylene-7β-propyl-19-nor-20-spirox-4-en-3-one 7α,18-dimethyl-15β,16β-methylene-19-nor-20-spirox-4-en-3-one 7β,18-dimethyl-15β,16β-methylene-19-nor-20-spirox-4-en-3-one 7α-ethyl-18-methyl-15β,16β-methylene-19-nor-20-spirox-4-en-3-one 7β-ethyl-18-methyl-15β,16β-methylene-19-nor-20-spirox-4-en-3-one 7α-ethenyl-18-methyl-15β,16β-methylene-19-nor-20-spirox-4-en-3-one 7β-ethenyl-18-methyl-15β,16β-methylene-19-nor-20-spirox-4-en-3-one 7α-cyclopropyl-18-methyl-15β,16β-methylene-19-nor-20-spirox-4-en-3-one 7β-cyclopropyl-18-methyl-15β,16β-methylene-19-nor-20-spirox-4-en-3-one 4,18-dimethyl-15β,16β-methylene-19-nor-20-spirox-4-en-3-one 4-chloro-18-methyl-15β,16β-methylene-19-nor-20-spirox-4-en-3-one 4,18-dimethyl-6β,7β,15β,16β-dimethylene-19-nor-20-spirox-4-en-3-one 4,18-dimethyl-6α,7α,15β,16β-dimethylene-19-nor-20-spirox-4-en-3-one 4-chloro-18-methyl-6β,7β,15β,16β-dimethylene-19-nor-20-spirox-4-en-3-one 4-chloro-18-methyl-6α,7α,15β,16β-dimethylene-19-nor-20-spirox-4-en-3-one.

7. Pharmaceutical products comprising at least one compound according to any of Claims 1 to 6 and a pharmaceutically acceptable carrier.

8. Pharmaceutical products according to Claim 7, additionally comprising at least one estrogen.

9. Pharmaceutical products according to Claim 8 comprising ethinylestradiol.

10. Pharmaceutical products according to Claim 8, comprising a natural estrogen.

11. Pharmaceutical products according to Claim 10, comprising estradiol.

12. Pharmaceutical products according to Claim 10, comprising estradiol valerate.

13. Pharmaceutical products according to Claim 10, comprising at least one conjugated estrogen.

## Revendications

1. 18-méthyl-15β,16β-méthylène-19-nor-20-spirox-4-én-3-one de formule générale I 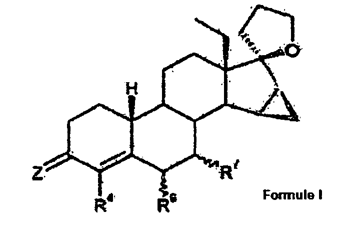 où
Z représente un atome d'oxygène, deux atomes d'hydrogène, un groupement =NOR ou =NNHSO₂R, où R représente un atome d'hydrogène ou un groupe alkyle linéaire ou ramifié comprenant respectivement 1 à 4 ou 3 à 4 atomes de carbone,
R⁴ représente un atome d'hydrogène, un atome d'halogène ou un groupe trifluorométhyle,
R⁶ et/ou R⁷ peuvent être en position α ou β et R⁶ et R⁷ signifient, indépendamment l'un de l'autre un atome d'hydrogène ou un groupe alkyle linéaire ou ramifié comprenant respectivement 1 à 4 ou 3 à 4 atomes de carbone ou un groupe alcényle linéaire ou ramifié comprenant respectivement 2 à 4 ou 3 à 4 atomes de carbone ou un groupe cycloalkyle saturé comprenant 3 à 5 atomes de carbone ou, ensemble, un groupe méthylène ou une double liaison.

2. 18-méthyl-15β,16β-méthylène-19-nor-20-spirox-4-én-3-one selon la revendication 1 de formule générale I, **caractérisée en ce que** Z représente un atome d'oxygène.

3. 18-méthyl-15β,16β-méthylène-19-nor-20-spirox-4-én-3-one selon la revendication 1 de formule générale I, **caractérisée en ce que** R⁴ représente un atome d'hydrogène, un groupe méthyle ou un atome de chlore.

4. 18-méthyl-15β,16β-méthylène-19-nor-20-spirox-4-én-3-one selon la revendication 1 de formule générale I, **caractérisée en ce que** R⁶ et R⁷ signifient ensemble un groupe 6α,7α-méthylène ou 6β,7β-méthylène ou une liaison supplémentaire.

5. 18-méthyl-15β,16β-méthylène-19-nor-20-spirox-4-én-3-one selon la revendication 1 de formule générale I, **caractérisée en ce que** R⁷ représente un groupe méthyle, éthyle, propyle ou éthényle en position α ou β ou un radical cyclopropyle en position α ou β.

6. Composés selon la revendication 1, à savoir la 18-méthyl-15β,16β-méthylène-19-nor-20-spiroxa-4,6-dién-3-one
la 18-méthyl-15β,16β-méthylène-19-nor-20-spirox-4-én-3-one
la 18-méthyl-6β,7β,15β,16β-diméthylène-19-nor-20-spirox-4-én-3-one
la 18-méthyl-6α,7α,15β,16β-diméthylène-19-nor-20-spirox-4-én-3-one
la 18-méthyl-15β,16β-méthylène-7α-propyl-19-nor-20-spirox-4-én-3-one
la 18-méthyl-15β,16β-méthylène-7β-propyl-19-nor-20-spirox-4-én-3-one
la 7α,18-diméthyl-15β,16β-méthylène-19-nor-20-spirox-4-én-3-one
la 7β,18-diméthyl-15β,16β-méthylène-19-nor-20-spirox-4-én-3-one
la 7α-éthyl-18-méthyl-15β,16β-méthylène-19-nor-20-spirox-4-én-3-one
la 7β-éthyl-18-méthyl-15β,16β-méthylène-19-nor-20-spirox-4-én-3-one
la 7α-éthényl-18-méthyl-15β,16β-méthylène-19-nor-20-spirox-4-én-3-one
la 7β-éthényl-18-méthyl-15β,16β-méthylène-19-nor-20-spirox-4-én-3-one
la 7α-cyclopropyl-18-méthyl-15β,16β-méthylène-19-nor-20-spirox-4-én-3-one
la 7β-cyclopropyl-18-méthyl-15β,16β-méthylène-19-nor-20-spirox-4-én-3-one
la 4,18-diméthyl-15β,16β-méthylène-19-nor-20-spirox-4-én-3-one
la 4-chloro-18-méthyl-15β,16β-méthylène-19-nor-20-spirox-4-én-3-one
la 4,18-diméthyl-6β,7β,15β,16β-diméthylène-19-nor-20-spirox-4-én-3-one
la 4,18-diméthyl-6α,7α,15β,16β-diméthylène-19-nor-20-spirox-4-én-3-one
la 4-chloro-18-méthyl-6β,7β,15β,16β-diméthylène-19-nor-20-spirox-4-én-3-one
la 4-chloro-18-méthyl-6α,7α,15β,16β-diméthylène-19-nor-20-spirox-4-én-3-one.

7. Préparations pharmaceutiques, contenant au moins un composé selon l'une quelconque des revendications 1 à 6 ainsi qu'un support pharmaceutiquement sans risque.

8. Préparations pharmaceutiques selon la revendication 7, contenant en outre au moins un estrogène.

9. Préparations pharmaceutiques selon la revendication 8 contenant de l'éthinylestradiol.

10. Préparations pharmaceutiques selon la revendication 8, contenant un estrogène naturel.

11. Préparations pharmaceutiques selon la revendication 10 contenant de l'estradiol.

12. Préparations pharmaceutiques selon la revendication 10 contenant du valérate d'estradiol.

13. Préparations pharmaceutiques selon la revendication 10, contenant au moins un estrogène conjugué.
